(19) Europäisches Patentamt
European Patent Office
Office européen des brevets

(11) Publication number: 0 031 606 B1

(12) EUROPEAN PATENT SPECIFICATION

(45) Date of publication of patent specification: 24.08.83

(21) Application number: 80201135.3

(22) Date of filing: 01.12.80

(51) Int. Cl.³: C 07 C 51/12, C 07 C 53/08, C 07 C 53/122, C 07 C 53/124, C 07 C 63/06, C 07 C 53/02, C 07 C 67/36, C 07 C 67/37, C 07 C 69/14, C 07 C 69/24, C 07 C 69/16

# (54) Process for the co-production of carboxylic acids and carboxylic acid esters.

(30) Priority: 21.12.79 GB 7944173

(43) Date of publication of application: 08.07.81 Bulletin 81/27

(45) Publication of the grant of the patent: 24.08.83 Bulletin 83/34

(84) Designated Contracting States: BE DE FR GB IT NL

(56) References cited:
DE - A - 2 303 271
GB - A - 2 029 409
US - A - 3 285 948
US - A - 4 101 450

JOURNAL OF THE AMERICAN CHEMICAL SOCIETY, vol. 100, no. 19, 13th September 1978, pages 6238—6240 G. BRACA et al.: "Simultaneous Carbonylation and Homologation of Dimethyl Ether and Homologation of Methyl Acetate to Ethyl Acetate in the Presence of Ruthenium Catalysts"

(73) Proprietor: SHELL INTERNATIONALE RESEARCH MAATSCHAPPIJ B.V.
Carel van Bylandtlaan 30
NL-2596 HR Den Haag (NL)

(72) Inventor: Drent, Eit
Badhuisweg 3
NL-1031 CM Amsterdam (NL)

(74) Representative: Puister, Antonius Tonnis, Mr. et al,
P.O. Box 302
NL-2501 CH The Hague (NL)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England

EP 0 031 606 B1

# Process for the co-production of carboxylic acids and carboxylic acid esters

The present invention relates to a process for the co-production of carboxylic acids and carboxylic acid esters from carboxylic acid esters having one carbon atom less in the molecule, carbon monoxide and hydrogen in the presence of a catalytic system. The invention relates in particular to a process for the co-production of acetic acid and ethyl acetate from methyl acetate under mild process conditions. Carboxylic acid esters produced according to the process according to the present invention are thus homologues of the carboxylic acid esters used as starting materials. The present invention also relates to a catalytic system which enables homologation to occur as well as to carboxylic acids and carboxylic acid esters obtained in accordance with the process according to the present invention. The present invention is of particular interest in that it allows the synthesis of carboxylic acid esters (together with carboxylic acids) from readily accessible carboxylic acid esters such as methyl acetate or ethyl acetate under mild process conditions.

The production of carboxylic acid esters via homologation has already been described in the literature. For instance, it is known from Dutch published patent application 7807520 that carboxylic acid esters (especially ethyl acetate from methyl acetate) can be prepared at elevated temperatures at a pressure of at least 100 bar, preferably between 200 and 1500 bar in the presence of cobalt, rhodium, ruthenium or iron or salts thereof. Apart from the fact that very high pressures have to be applied in order to get a reasonable conversion, the process as described in the Dutch published patent application 7807520, also has the disadvantage that water is produced as the co-product. It will be clear that water can cause a substantial hydrolysis of the esters present in the reaction mixture (whether being present as product or as starting material). Even when very high, unattractive pressures (e.g. well over 1000 bar) are used substantial amounts of alkanols are produced.

It is further known from German Offenlegungsschrift 2733663 that the homologation of methyl acetate (or of its precursor dimethyl ether) can be carried out using a ruthenium carbonyl compound and an iodide or bromide promoter at elevated temperatures and pressures. The process as described in the German Offenlegungsschrift also has to be carried out at very high pressures (well over 200 bar) and substantial amounts of other products (including not only alkanols but also methane) are formed.

It is known from Dutch published patent application 7602096 that methyl acetate can be converted using carbon monoxide and hydrogen in the presence of a specific catalyst comprising a Group VIII noble metal compound and a halogen (especially iodine) source into acetic acid and ethylidene diacetate. However, ethyl acetate (the homologation product according to the process according to the present invention) is not even mentioned as a by-product.

It has now been found that carboxylic acids and carboxylic acid esters can be co-produced from carboxylic acid esters having one carbon atom less in the molecule, carbon monoxide and hydrogen under very mild process conditions using a specific catalytic system.

The present invention relates to a process for the co-production of carboxylic acids according to the general formulae $R^1$—COOH and $R^2$—COOH and carboxylic acid esters according to the general formulae $R^1$—$COOCH_2R^2$ and $R^2$—$COOCH_2R^1$ wherein the groups $R^1$ and $R^2$ which may be the same or different each represent an alkyl group having from 1 to 20 carbon atoms which may be substituted by fluorine or chlorine or represent an aryl, alkaryl or aralkyl group which may be substituted by fluorine or chlorine whilst $R^1$ may also represent a hydrogen atom, characterized in that a carboxylic acid ester according to the general formula $R^1$—$COOR^2$ and/or an ether according to the general formula $R^3OR^4$, wherein $R^1$ and $R^2$ are as defined hereinbefore and $R^3$ and $R^4$, which may be the same or different each represent an alkyl group having from 1 to 20 carbon atoms which may be substituted by fluorine or chlorine or represents an aryl, alkaryl or aralkyl group which may be substituted by fluorine or chlorine is reacted with carbon monoxide and hydrogen at elevated temperature and pressure in the presence of a catalytic system which comprises a ruthenium compound, a Group II metal iodide and/or bromide or a transition-metal iodide and/or bromide and a further Group VIII metal compound.

It should be noted that the composition of the reaction product mixture will be governed by the choice of the starting carboxylic acid esters and/or ethers. For instance, when starting materials are used, wherein the groups $R^1$ and $R^2$ are identical, such as in methyl acetate, dimethyl ether and ethyl propionate, the reaction product mixture will normally contain only the carboxylic acid ester homologue and the appropriate acid. When starting materials are used wherein the groups $R^1$ and $R^2$ are not identical, a more complex reaction product mixture will be obtained which comprises normally at least two carboxylic acid ester homologues and two appropriate carboxylic acids. For instance, when ethyl acetate is used as the starting material the reaction product mixture comprises propyl acetate, ethyl propionate, propionic acid and acetic acid.

It will be appreciated that any carboxylic acid ester homologue produced according to the present process can serve as starting material in the process according to the present invention thus forming the next carboxylic acid ester homologue(s) and the appropriate carboxylic acid(s).

Since also carboxylic acids are produced according to the process according to the present invention, it cannot be excluded that transesterification reactions, i.e. reactions between carboxylic acid(s) produced and (starting) carboxylic acid esters or between different carboxylic acid esters, may also occur under the prevailing reaction conditions. It will be clear that transesterification reactions do

not alter the product composition when the starting material comprises compounds wherein $R^1$ and $R^3$ are identical, but may alter the product composition when the groups $R^1$ and $R^2$ are not identical.

For the purpose of the present invention carboxylic acids and carboxylic acid esters whether obtained via a further homologation of produced carboxylic acid ester or by virtue of a transesterification process under the prevailing conditions are considered to be within the scope of the present invention.

From the above it will be clear that preference is given to processes wherein starting materials are used wherein the groups $R^1$ and $R^2$ are identical since a less complex reaction mixture will be obtained. The process according to the present invention is of special interest for the co-production of acetic acid and ethyl acetate from methyl acetate according to the equation:

$$2\ CH_3COOCH_3 + 2\ CO + 2\ H_2 \rightarrow CH_3COOC_2H_5 + 2\ CH_3COOH$$

since the products can be obtained with high selectivity and close to the stoichiometrically expected ratio. This is of special interest when the process according to the present invention is part of an integrated process, wherein acid produced — for instance acetic acid — is to be recycled in the process. Moreover, the process according to the present invention can be carried out conveniently at surprisingly low pressures, e.g. pressures well below 100 bar can be used advantageously.

The present invention also relates to a catalytic system which enables homologation to occur which comprises a ruthenium compound, a Group II metal iodide and/or bromide or a transition metal iodide and/or bromide and a further Group VII metal compound.

Starting materials which can be used conveniently in the process according to the present invention for the production of acid(s) and ester homologues corresponding with the starting ester(s) comprise compounds according to the general formulae $R^1$—$COOR^2$ and/or $R^3OR^4$, wherein $R^1$, $R^2$, $R^3$, and $R^4$ which may be the same or different, each represent an alkyl group having from 1 to 12 carbon atoms, or an aryl, alkaryl or aralkyl group having up to 12 carbon atoms whilst $R^1$ may also represent a hydrogen atom. Preference is given to the use of compounds according to the general formulae $R^1$—$COOR^2$ and/or $R^3OR^4$, wherein $R^1$ $R^2$, $R^3$ and $R^4$ are the same and represent an alkyl group having from 1 to 12 carbon atoms or an aryl, alkaryl or aralkyl group having up to 12 carbon atoms. Most preferred starting materials are methyl acetate and dimethyl ether.

When ethers according to the general formula $R^3OR^4$ are used as starting materials in the process according to the present invention, it would appear that these compounds will be converted primarily into the corresponding esters by the introduction of a carbon monoxide moiety into the molecule which molecule may then be subjected to the homologation reaction according to the present invention. If desired, the reaction according to the present invention can be carried out in two stages when an ether is used as the starting material. Firstly, the ether will be converted into the corresponding ester which in its turn, in the same or in a different vessel, will be converted into the final products. Also mixtures of carboxylic acid esters and/or ethers can be used as starting materials.

It has been found that the catalytic system to be used in the process according to the present invention is rather critical in that the presence of a ruthenium compound, a Group II metal iodide and/or bromide or a transition metal iodide and/or bromide and a further group VIII metal compound is mandatory for the selective and rapid co-production of esters and acids.

For instance, from comparative Example A it will be clear that when carrying out the process according to the present invention but omitting the ruthenium compound, only a small amount of ester will be produced together with a large excess of the corresponding acid. Using a rhodium trichloride-methyl iodide catalytic system (comparable with the reaction disclosed in Dutch published patent application 7602096) under the mild reaction conditions according to the present invention no ester could be found but a fair amount of ethylidene diacetate was obtained together with acetic acid (see comparative Example B).

Ruthenium compounds which can be used conveniently in the process according to the present invention comprise ruthenium (III) chloride, ruthenium (III) chloride trihydrate, ruthenium (IV) chloride, ruthenium (III) bromide, the ruthenium oxides organic ruthenium salts such as ruthenium (III) formate, ruthenium (III) acetate, ruthenium (III) propionate, ruthenium (III) butyrate, ruthenium pentacarbonyl, trirutheniumdodecacarbonyl and mixed ruthenium halocarbonyls such as bis-(rutheniumtricarbonyl-dibromide), and other organoruthenium complexes.

Further Group VIII metal compounds which can be used together with a ruthenium compound as described hereinabove comprise especially rhodium and palladium compounds although other Group VIII metal compounds can also be used. Examples of suitable rhodium compounds comprise rhodium oxide, rhodium (III) hydroxide, rhodium (III) chloride, rhodium (III) chloride trihydrate, rhodium (III) bromide, rhodium (III) iodide as well as the corresponding pyridine and phosphine complexes such as tris(pyridine) rhodium (III) chloride or dichloro bis-(triphenylphosphine) rhodium, rhodium (III) formate, rhodium (III) acetate, rhodium (III) butyrate, rhodium (III) naphthenate, dirhodium octacarbonyl, tetrarhodium dodecacarbonyl, hexarhodium hexadecacarbonyl, rhodium dicarbonylacetylacetonate and other organorhodium complexes. Preference is given to the use of rhodium (III) chloride trihydrate.

Examples of suitable palladium compounds comprise palladium chloride, palladium chloride

dihydrate, palladium bromide, palladium iodide, palladium oxide, or an organic palladium salt or complex such as palladium formate, palladium acetate, palladium butyrate and palladium acetylacetonate Preferred palladium compounds to be used in the process according to the present invention are palladium chloride, palladium chloride dihydrate and palladium acetate.

The ratio of ruthenium compound and further Group VIII compound to be applied is not critical and can vary between wide limits, e.g. between 20:1 and 1:20. Preference being given to the use of the compounds in a ratio between 5:1 and 1:5. Good results can be obtained using the ruthenium compound in half the molar amount of the further Group VIII metal employed.

The amount of ruthenium compound and further Group VIII compound to be used is not critical and any amount which exerts catalytic activity can be used. Amounts as low as 0.001%w, calculated on carboxylic acid ester or ether to be converted can be used preference being given to amounts in the range of from 0.01—10%w, most preferably between 0.05—5%w.

Metal iodides and/or bromides which can be used comprise Group II iodides and/or bromides as well as transition metal iodides and/or bromides. Examples of metal compounds which can be conveniently used comprise magnesium bromide and/or iodide, chromium (III) iodide, cobalt (II) iodide, cobalt (II) bromide, nickel (II) iodide, nickel (II) bromide, copper (II) iodide, copper (II) bromide, zinc iodide and zinc bromide. Preferred metal iodides and bromides to be used are cobalt (II) iodide, zinc iodide and zinc bromide. Normally iodides are preferred over the corresponding bromides.

It should be noted that use can also be made of combinations of metal salts which can give rise to in situ formation of Group II iodides or — bromides or transition metal iodides or bromides, e.g. a mixture of zinc acetate and an alkali (ne earth) metal iodide or bromide.

The amount of metal iodide and/or metal bromide to be used can also vary between wide limits. In general, the use of an excess of metal iodide and/or metal bromide over the other metal compounds in the catalytic system is preferred. Suitable molar ratios of metal iodide and/or metal bromide to other metal compounds are in the range of from 200 to 0.1 ratios in the range of from 50 to 10 being preferred.

It has been found that other iodine-containing compounds such as the alkyliodides, in particular methyliodide, can be used in addition to the metal iodide and/or metal bromide as defined hereinbefore. The amount of additional iodine-containing compound is not critical. Normally the additional iodine-containing compound will be used in about the same amount as the metal iodide applied.

It should be noted however, that the mere presence of an alkyl iodide, such as methyliodide in the absence of a metal iodide and/or metal bromide as defined hereinbefore is insufficient under otherwise similar reaction conditions to obtain the carboxylic acid ester and the acid in the stoichiometrically expected ratio. From comparative Example B it will be clear that under such conditions acetic acid is produced in much to high an amount.

If desired, the process according to the present invention can be carried out in the additional presence of organic compounds which are capable of forming a co-ordination compound with the Group VIII metal moieties present in the catalytic system according to the present invention. Suitable compounds comprise organo-phosphorous, organo-arsenic, organic-antimony, organo-nitrogen, organo-sulphur and organo-oxygen compounds. Preferred compounds having co-ordinative properties are organo-phosphorus compounds and organo-nitrogen compounds. Also compounds containing both phosphorus and oxygen atoms or nitrogen and oxygen atoms can be suitably applied.

Examples of organo-nitrogen compounds which can be used conveniently comprise pyrrole, alkyl-substituted pyrroles, pyrrolidine, alkyl-substituted pyrrolidines, pyridine, alkyl-substituted pyridines, piperidines, alkyl-substituted piperidines, pyrimidine, alkyl-substituted pyrimidines, pyrazine, benztriazole, tetraethylenediamine, 1,10-phenantroline, alkyl-substituted 1,10-phenantrolines, morpholine and alkyl-substituted morpholines. Preference is given to the use of pyridine and alkyl-substituted pyridines such as the various picolines, e.g. alphapicoline.

Examples of organo-phosphorus compounds which can be used conveniently comprise tertiary phosphines according to the general formula $PR^5R^6R^7$, wherein $R^5$, $R^6$ and $R^7$, which may be the same or different, each represent an alkyl, cycloalkyl or aryl group having up to 10 carbon atoms. Preferred organo-phosphorus compounds comprise trimethyl-phosphine, triethylphosphine, tri-n-butylphosphine and triphenyl-phosphine. Also the corresponding organo-phosphites according to the general formula $OPR^5R^6R^7$ (wherein $R^5$, $R^6$ and $R^7$ are as defined herein-before) can be used as co-ordination compounds.

The amount of organic compound additionally to be used in the process according to the present invention is generally related to the amount of Group VIII metal compounds present in the reaction mixture. Higher as well as lower amounts can also be used. Therefore amounts ranging of from 0.1—100 times the total molar amount of Group VIII metal compounds present can be suitably applied. Preference is given to amounts up to 10 times the molar amount of Group VIII metal compounds present. It should be stated however, that the presence of such co-ordination compounds is optional.

The process according to the present invention can be carried out using a wide range of temperatures. Temperatures up to 300°C can be suitably applied. Preference is given to temperatures

in the range of from 50°C to 200°C, most preferred temperatures are in the range between 125°C and 175°C.

The process according to the present invention can be carried out using low pressures, e.g. pressures as low as 5 bar. Pressures in the range of from 20 to 100 bar are preferred. Higher pressures, e.g. pressures as high as 1000 bar can be applied, but they do not contribute substantially whilst the investment and energy costs involved increase considerably without compensating substantially in terms of product yield and/or selectivity.

According to the reaction equation carbon monoxide and hydrogen are consumed in a molar ratio of 1:1. It has been found, however, that without any substantial disadvantage wider molar ratios, e.g. ratios of from 1:10 to 10:1 can be applied. Preference is given to ratios carbon monoxide:hydrogen being in the range of from 1:0.5 to 1:2.

The reaction time is not critical and will depend largely on the temperature and pressure applied. Reaction times of from 1 to 20 hours are sufficient, preference being given to reaction times in the range of from 5 to 15 hours. Shorter or longer reaction times are not excluded.

The process according to the present invention can also be carried out in the presence of a solvent. Suitable solvents comprise carboxylic acids such as acetic acid or propanoic acid; carboxylic acid esters, such as methyl acetate, ethyl acetate, methylpropionate or ethyl propionate (being used as solvent as well as starting material), and cyclic ethers such as tetrahydrofuran, 1,4-dioxane, 1,3-dioxane and the dioxolanes. Also linear or branched dialkyl ethers used in excess as starting material may be regarded as solvent for the process according to the present invention. Suitable dialkylethers comprise dimethyl ether, diethyl ether and methyl t-butyl ether.

Other compounds which can be used as solvent in the process according to the present invention comprise (a) cyclic sulphones and sulphoxides. Examples of such compounds are dimethylsulphone, sulpholane, 2-methyl sulpholane, 3-methyl sulpholane, dimethylsulphoxide and diethyl sulphoxide.

Good results can be obtained when carboxylic acids such as acetic acid are used as solvent. The amount of solvent which can be used in the process is not critical and can vary between wide limits. Volumes of solvent as high as 50 times the volume of the reagents can be suitably applied.

It has been found that the mild conditions according to the present invention even tolerate the presence of some water in the reaction medium. Although the presence of water is not preferred, amounts of up to 15%w, based on total solvent, can be applied.

The process according to the present invention can be carried out in the liquid phase as well as in the gaseous phase. Preference is given to a liquid phase which enables a convenient introduction of carbon monoxide and hydrogen into the reaction vessel. If desired, the carbon monoxide and hydrogen can be introduced together into the reaction vessel. The process according to the present invention can be carried out batchwise, semi-continuously or continuously. The reaction may comprise one or more autoclaves or one or more reactor tubes which walls are made of or coated with inert materials.

The process according to the present invention is also of interest in that it can be integrated with known processes, either for the production of the starting materials (i.e. carboxylic acid esters or the corresponding ethers) or for the conversion of the carboxylic acid esters produced into other products, e.g. by transesterification processes. For instance, when the present process produces ethyl acetate, it can be integrated with a process for the preparation of methyl acetate from acetic acid and methanol using an acidic catalyst. Since the present process produces also acetic acid, that comound can be (partially) recycled to serve as feedstock for the preparation of methyl acetate. If desired, the present process can also be integrated with a transesterification process, wherein ethylacetate is transesterified with methanol to give methyl acetate (which can be recycled to serve as (partial) feedstock for the present process) and ethanol which can either be sold as such or converted into other products such as ethylene. In such a case acetic acid and/or methyl acetate can be removed from the system in an amount equimolar with ethanol produced. From the above, it will be clear that the net chemical reaction when combining the present process and the preparation of methyl acetate from methanol and acetic acid amounts to the conversion of methanol using carbon monoxide and hydrogen into ethyl acetate and water. When also the transesterification as described hereinbefore is taken into account the net reaction amounts to the conversion of methanol using carbon monoxide and hydrogen into methyl acetate and/or acetic acid, ethanol and water.

The reaction products may be worked up by techniques known in the art. For instance, the reaction product mixture may be subjected to a (fractional) distillation to separate the carboxylic acid ester(s) and the acid(s) produced. If desired further purification treatment can be given to one or both separated products.

The products obtained by the process according to the present invention can either be used as such, e.g. as solvent or as reagent, or can be used as intermediates in further chemical reactions, e.g. ethyl acetate can be hydrolyzed using known techniques to ethanol.

The present invention will now be illustrated by means of the following non-limiting Examples.

Example I

The experiment was carried out in a 300 ml magnet-driven autoclave of Hastelloy C which contained 0.6 mol methyl acetate, 1 mmol rhodium (III) chloride trihydrate, 0.5 mmol ruthenium (III)

chloride trihydrate, 30 mmol zinc iodide and 3 mmol alphapicoline. The vessel was flushed with carbon monoxide and further charged with carbon monoxide (15 bar partial pressure) and hydrogen (partial pressure 30 bar). The autoclave was then heated to 155°C and kept at this temperature for 5 hours. Carbon monoxide had been converted almost completely and the reaction mixture contained as determined by gas-liquid chromatography 16.1%w ethyl acetate, 21.6%w acetic acid and a very small amount of ethylidene diacetate (0.1%w), the remainder primarily being unconverted methyl acetate On a molar basis the conversion of the starting material was about 30% with an almost 100% selectivity towards the two products in the desired ester/acid molar ratio of 1:2.

Example II

The experiment as described in Example I was repeated with the exception that the reaction was carried out in the absence of alpha-picoline for a period of 15 hours. Again the amount of carbon monoxide originally present had been converted almost completely. The reaction mixture contained as products (determined by gas-liquid chromatography) 15.1%w ethylacetate and 19.7%w acetic acid. No ethylidene diacetate could be detected.

Example III

The experiment as described in Example I was repeated using an initial carbon monoxide partial pressure of 20 bar and an initial hydrogen partial pressure of 20 bar. The reaction mixture was heated for 15 hours at 155°C. Again the amount of carbon monoxide originally present had been converted almost completely. The reaction mixture contained 15.8%w ethyl acetate; 21.9%w acetic acid and 2.2%w ethylidene diacetate. The desired products (ethyl acetate and acetic acid) had been obtained in the desired 1:2 ratio.

Example IV

A 300 ml magnet-driven autoclave made from Hastelloy C was charged with 0.6 mol methyl acetate, 0.7 mmol palladium acetate, 0.35 mmol ruthenium (III) chloride trihydrate, 0 mmol zinc iodide, 14 mmol methyl iodide and 4 mmol triphenylphosphine. The vessel was further charged with carbon monoxide (initial pressure 15 bar) and hydrogen (initial pressure 30 bar). The vessel was heated to 135°C and kept at this temperature for 15 hours. Again, the carbon monoxide originally present had been converted almost completely. The reaction mixture contained 13.7%w ethylacetate, 18.7%w acetic acid and 0.1%w ethylidene diacetate.

Example V

The autoclave described in the previous Example was charged with 0.6 mol ethylacetate as the starting material, 1 mmol rhodium (III) chloride, 0.5 mmol ruthenium (III) chloride trihydrate, 30 mmol zinc iodide and 3 mmol alphapicoline. The autoclave was further charged with carbon monoxide (initial pressure 15 bar) and hydrogen (initial pressure 30 bar). The vessel was heated at a temperature of 170°C for 15 hours. Again, the carbon monoxide originally present had been converted almost completely. The reaction mixture contained a mixture of esters and acids. The composition of the products in reaction mixture as determined by gas-liquid chromatography was as follows:

ethylpropionate 15.6%w
propylacetate 4.8%w
propionic acid 6.0%w
acetic acid 15.1%w

Example VI

The experiment described in the previous Example was repeated using ethylpropionate as the starting material. The reaction was carried out at 155°C during 5 hours. The reaction mixture contained 10.9%w propyl propionate and 14.9%w propionic acid. Again, the desired products had been obtained in the desired 1:2 (ester:acid) ratio.

Example VII

The experiment described in the previous Example was repeated using methyl propionate as the starting material. The reaction was carried out at 160°C during 5 hours. The composition of the products in the reaction mixture was as follows:

ethyl propionate 3.4%w
propyl acetate 5.0%w
methyl acetate 4.8%w
propyl propionate 9.6%w
acetic acid 9.4%w
propionic acid 21.5%w

Also a trace of ethylacetate was detected.

Example VIII

The experiment described in the previous Example was repeated using isopropyl isobutyrate as the starting material. The composition of the products in the reaction mixture was 7.1% isobutylisobutyrate, 15.1%w n-butylisobutyrate and 25.4% isobutyric acid.

Example IX

The experiment described in the previous Example was repeated using methyl benzoate as the starting material. The reaction was carried out at 165°C during 15 hours. Ethylbenzoate (6%w) and significant quantities of acetic acid and solid benzoic acid were obtained in fair yields.

Example X

The experiment described in Example VIII was repeated using n-propylpropionate as the starting material. The composition of the products in the reaction mixture was as follows:

propyl butyrate 7.0%w
butyl propionate 3.2%w
propionic acid 8.8%w
butyric acid 2.3%w

Also traces of butylbutyrate and isobutyric acid were detected.

Example XI

a) The autoclave described in Example III was charged with 20 g methyl acetate as the starting material, 1 mmol rhodium (III) chloride trihydrate, 0.5 mmol ruthenium (III) chloride trihydrate and 30 mmol zinc iodide. Acetic acid (25 ml) was used as a solvent. The vessel was further charged with carbon monoxide (initial partial pressure 20 bar) and hydrogen (initial partial pressure 20 bar) and then heated for 5 hours at 160°C whilst carbon monoxide and hydrogen were added continuously at a total pressure of 60 bar. From gas-liquid chromatography it appeared that methyl acetate had been converted at a rate of about 35 g/g rhodium/hr (total conversion 85%). The reaction product mixture contained 20%w ethyl acetate.

b) The experiment described in Example XI[a] was repeated using 25 ml acetic acid and 2.5 ml water as the solvent mixture. The experiment was carried out for 15 hours at 155°C using a carbon monoxide:hydrogen ratio of 1:1. From gas-liquid chromatography it appeared that 94% of the starting material had been converted. The reaction product mixture contained 15.2%w ethyl acetate and 7.4%w propionic acid. The presence of ethanol was not observed.

c) The experiment described in Example XI[a] was repeated using sulfolane (25 ml) as the solvent. The reaction was carried out for 15 hours at 155°C at a carbon monoxide:hydrogen ratio of 1:1. The reaction product mixture contained 9.7%w ethylacetate and 13.6%w acetic acid. The conversion rate of methyl acetate was about 10 g/g rhodium/hr.

Example XII

The experiment described in Example I was repeated using a mixture of methyl acetate (20 g) and dimethyl ether (17 g) as the starting material. The reaction was carried out at 155°C during 5 hours using an initial carbon monoxide partial pressure of 15 bar and an initial hydrogen partial pressure of 30 bar. The conversion rate was about 40 g/g rhodium/hr (including dimethyl ether). The reaction product mixture contained 9.7%w ethyl acetate and 14.0%w acetic acid and 70%w methylacetate.

Example XIII

a) The autoclave described in Example XII was charged with methyl formate (0.66 mol), 0.1 mmol rhodium (III) chloride trihydrate, 1 mmol ruthenium (III) chloride trihydrate, 40 mmol zinc iodide and 3 mmol alpha-picoline. The reaction was carried out at 155°C during 15 hrs using an initial carbon monoxide partial pressure of 15 bar and an initial hydrogen partial pressure of 30 bar. The reaction product mixture contained 39%w methyl acetate, 4.6%w ethyl acetate, 6.0%w acetic acid and a considerable quantity of formic acid together with water and carbon dioxide (decomposition products of formic acid).

b) The experiment described in Example XIII[a] was repeated using half the amount of zinc iodide. The reaction was performed at a carbon monoxide:hydrogen ratio of 1:2. It appears that a total pressure of 12 bar carbon monoxide/hydrogen had been consumed. The reaction product mixture contained 23.8%w methyl acetate, 1.5%w ethyl acetate, 12.9%w acetic acid, 8.7%w formic acid and 5.0%w water (decomposition of formic acid).

Comparative Example A

The experiment described in this Comparative Example A was carried out by charging the 300 ml magnet-driven autoclave with 0.6 mol methyl acetate, 1 mmol rhodium (III) chloride trihydrate, 30 mmol zinc iodide and 3 mmol alpha-picoline. The autoclave was further charged with carbon monoxide

(initial pressure 15 bar) and hydrogen (initial pressure 30 bar). The reaction vessel was kept at 155°C during 15 hours. From gas-liquid chromatography it appeared that 28.8%w acetic acid had been formed but only 8.8%w ethyl acetate. In the absence of a ruthenium compound, the desired ratio ester: acid = 1:2 was not reached at all. By slightly changing the original pressures (i.e. starting with an initial carbon monoxide pressure of 20 bar and an initial hydrogen pressure of 20 bar) and at a temperature of 160°C under otherwise similar conditions, even more acetic acid had been formed (39.6%w) whereas the amount of ethyl acetate produced had been halved (4.5%w). Also 1.7%w ethylidene diacetate appeared to be present in the reaction mixture.

Comparative Example B

The experiment described in Comparative Example A was repeated with the exception that also 0.5 mmol ruthenium (III) chloride trihydrate was present and that zinc iodide had been replaced by 60 mmol methyliodide. The reaction was performed for 5 hours at 155°C. The reaction mixture as determined by gas-liquid chromatography contained a large amount of acetic acid (31.8%w) and by-products were produced in a larger amount than ethylacetate: (ethylidene diacetate 0.7%w, ethanol 2.0%w, dimethylether 4%w and propionic acid 5.3%w compared with 7.1%w ethyl acetate).

Comparative Example C

The experiment described in Comparative Example A was repeated with the exception that zinc iodide had been replaced with methyliodide (30 mmol). The reaction was carried out at 150°C during 15 hours starting from an initial carbon monoxide pressure of 20 bar and an initial hydrogen pressure of 20 bar. The reaction mixture did not contain ethyl acetate but contained acetic acid (15.7%w) as well as a fair amount of ethylidene diacetate (9%w).

Comparative Example D

The 300 ml magnet-driven autoclave made from Hastelloy C was charged with 0.6 mol methyl acetate, 0.7 mmol palladium acetate, 0.35 mmol ruthenium (III) chloride trihydrate, 14 mmol methyl iodide and 4 mmol triphenylphosphine. The reaction was carried out at 135°C during 15 hours starting at an initial carbon monoxide pressure of 20 bar and an initial hydrogen pressure of 20 bar. Only a trace of ethyl acetate (0.8%w) could be detected in the reaction mixture using gas-liquid chromatography. The main products obtained were acetic acid (7.9%w) and ethylidene diacetate (6.3%w).

Comparative Example E

The autoclave referred to in Comparative Example D was charged with 0.6 mol methyl acetate, 1 mmol rhodium (III) chloride trihydrate, 20 mmol zinc acetate, 40 mmol methyl iodide and 3 mmol alpha-picoline. The autoclave was further charged with carbon monoxide (initial pressure 20 bar) and hydrogen (initial pressure 20 bar). The reaction was carried out at 150°C for 15 hours. The reaction mixture contained a fair amount of acetic acid (25.5%w) but only a small amount of ethylacetate (3.3%w) and even more ethylidene diacetate (4.0%w). This experiment indicates that the combined presence of methyliodide and a zinc salt in the absence of a ruthenium compound is insufficient to obtain the desired product ratio. When under otherwise similar conditions a double amount of zinc acetate was used the production of acetic acid dropped considerably to 8.9%w and only a small increase in the production of ethyl acetate to 3.9%w was found.

Comparative Example F

The experiment described in Comparative Example A was repeated, with the exception that 1 mmol ruthenium (III) chloride trihydrate was used instead of 1 mmol rhodium (III) chloride trihydrate. From gas-liquid chromatography it appeared that 3.5%w acetic acid had been formed and 2.5%w ethyl acetate 0.5%w dimethylether and 0.2%w ethanol being the main by-products. This Comparative Example clearly demonstrates the absence of a beneficial effect when a further group VIII metal compound is not present.

Comparative Example G

a) The experiment described in Comparative Example B was repeated using 56 mmol lithium iodide instead of methyliodide. The reaction was performed at 155°C during 5 hours at an initial carbon monoxide partial pressure of 15 bar and an initial hydrogen partial pressure of 30 bar. The reaction product mixture contained a large amount of acetic acid (64%w). Ethyl acetate had been produced in 11.9%w, thus giving an ester:acid ratio of 1:7. Some ethanol (about 0.5%w) had also been produced.

b) The experiment described in Comparative Example Ga was repeated using half the amount of lithium iodide. Having applied otherwise similar conditions, the reaction mixture contained 7.5%w ethyl acetate, 25%w acetic acid and ethylidene diacetate had been co-produced in a fair amount (about 4.6%w). The ester and acid had been produced in a 1:5 ratio.

c) The experiment described in Comparative Example Ga was repeated by heating the reaction mixture at 135°C for 5 hours. The reaction mixture contained 7.8%w ethyl acetate and 49%w acetic acid. The ester and the acid had even been produced in a 1:9 ratio.

## Claims

1. A process for the co-production of carboxylic acids according to the general formulae $R^1$—COOH and $R^2$—COOH and carboxylic acid esters according to the general formulae $R^1$—$COOCH_2R^2$ and $R^2$—$COOCH_2R^1$, wherein the groups $R^1$ and $R^2$ which may be the same or different each represent an alkyl group having from 1 to 20 carbon atoms which may be substituted by fluorine or chlorine or represent an aryl, alkaryl or aralkyl group which may be substituted by fluorine or chlorine whilst $R^1$ may also represent a hydrogen atom, characterized in that a carboxylic acid ester according to the general formula $R^1$—$COOR^2$ and/or an ether according to the general formula $R^3OR^4$ wherein $R^1$ and $R^2$ are as defined hereinbefore and $R^3$ and $R^4$ which may be the same or different each represent an alkyl group having from 1 to 20 carbon atoms which may be substituted by fluorine or chlorine or represent an aryl, alkaryl or aralkyl group which may be substituted by fluorine or chlorine, is reacted with carbon monoxide and hydrogen at elevated temperature and pressure in the presence of a catalytic system which comprises a ruthenium compound, a Group II metal iodide and/or bromide or a transition metal iodide and/or bromide and a further Group VIII metal compound.

2. A process according to claim 1 characterized in that as starting materials are used compounds according to the general formulae $R^1$—$COOR^2$ and/or $R^3OR^4$ wherein $R^1$, $R^2$, $R^3$ and $R^4$ which may be the same or different, each represent an alkyl group having from 1 to 12 carbon atoms or an aryl, alkaryl or aralkyl group having up to 12 carbon atoms whilst $R^1$ may also represent a hydrogen atom, and preferably are the same and represent an alkyl group having of from 1 to 12 carbon atoms or an aryl, alkaryl or aralkyl group having up to 12 carbon atoms.

3. A process according to claim 1 or 2 characterized in that as ruthenium compound is used ruthenium (III) chloride, ruthenium (III) chloride trihydrate, ruthenium (IV) chloride, ruthenium (III) bromide, ruthenium oxide or an organic ruthenium salt or complex.

4. A process according to any one of the preceding claims characterized in that as further Group VIII metal compound is used a rhodium compound such as rhodium oxide, rhodium (III) hydroxide, rhodium (III) chloride, rhodium (III) chloride trihydrate, rhodium (III) bromide, rhodium (III) iodide or an organic rhodium salt or complex, preferably rhodium (III) chloride trihydrate, or a palladium compound such as palladium chloride, palladium chloride dihydrate, palladium bromide, palladium iodide, palladium oxide or an organic palladium salt or complex, preferably palladium chloride, palladium chloride dihydrate or palladium acetate.

5. A process according to any one of the preceding claims, characterized in that the ruthenium compound and the further Group VIII metal compound are used in a ratio between 20:1 and 1:20, preferably between 5:1 and 1:5 and in an amount of at least 0.001%w and preferably in an amount in the range of from 0.01—10%w, calculated on carboxylic acid ester and/or ether to be converted.

6. A process according to any one of the preceding claims, characterized in that the catalytic system comprises one or more of the compounds magnesium bromide and/or iodide, chromium (III) iodide, cobalt (II) iodide, cobalt (II) bromide, nickel (II) iodide, nickel (II) bromide, copper (II) iodide, zinc iodide and zinc bromide, and preferably cobalt (II) iodide, zinc iodide or zinc bromide.

7. A process according to any one of the preceding claims characterized in that additionally an organo-phosphorus compound such as tertiary phosphine or phosphite, especially tri-n-butyl phosphine or triphenylphosphine, an organo-arsenic compound, an organo-antimony compound, an organo-nitrogen compound such as pyridine or an alkyl-substituted pyridine, especially alpha-picoline, an organic-sulphur compound or an organic-oxygen compound is present in the reaction mixture which compound is capable of forming a co-ordination compound with a Group VIII metal moiety present in the catalytic system.

8. A process according to any one of the preceding claims characterized in that the reaction is carried out at a temperature up to 300°C, preferably at a temperature in the range of from 50°C to 200°C, and especially between 125°C and 175°C.

9. A process according to any one of the preceding claims characterized in that the process is carried out using a low pressure, preferably a pressure between 20 and 100 bar.

10. A process according to any one of the preceding claims characterized in that the reaction is carried out in the presence of a solvent such as a carboxylic acid, a carboxylic acid ester, a cyclic ether, an (a) cyclic sulphone such as dimethyl sulphone, sulfolane, 2-methylsulfolane or 3-methylsulfolane or a sulphoxide such as dimethylsulphoxide or diethylsulphoxide.

11. A process according to any one of the preceding claims, characterized in that as starting material is used a carboxylic acid ester which has been obtained by reacting an alkanoic acid and an alkanol, especially acetic acid and methanol, in the presence of an acidic catalyst and wherein optionally the alkanoic acid produced, especially acetic acid, is recycled to the starting material and the carboxylic acid ester obtained is subjected to a trans-esterification process.

12. A catalytic system characterized by a ruthenium compound, especially ruthenium (III) chloride trihydrate, a further Group VIII metal compound, especially a rhodium compound or a palladium compound, and a Group II metal iodide and/or bromide or a transition metal iodide and/or bromide, especially zinc iodide.

**Patentansprüche**

1. Ein Verfahren zur gleichzeitigen Herstellung von Carbonsäuren gemäß den allgemeinen Formeln $R^1$—COOH und $R^2$—COOH und Carbonsäureestern gemäß den allgemeinen Formeln $R^1$—$COOCH_2R^2$ und $R^2$—$COOCH_2R^1$, in denen die Gruppen $R^1$ und $R^2$, die gleich oder verschieden sein können, je eine Alkylgruppe mit 1 bis 20 Kohlenstoffatomen darstellen, die mit Fluor oder Chlor substituiert sein kann, oder eine Aryl-, Alkylaryl- oder Arylalkylgruppe darstellen, die mit Fluor oder Chlor substituiert sein kann, während $R^1$ auch ein Wasserstoffatom darstellen kann, dadurch gekennzeichnet, daß ein Carbonsäureester gemäß der allgemeinen Formel $R^1$—$COOR^2$ und/oder ein Äther gemäß der allgemeinen Formel $R^3OR^4$, in denen $R^1$ und $R^2$ wie oben definiert sind und $R^3$ und $R^4$, die gleich oder verschieden sein können, je eine Alkylgruppe mit 1 bis 20 Kohlenstoffatomen darstellen, die mit Fluor oder Chlor substituiert sein kann, oder eine Aryl-, Alkylaryl- oder Arylalkylgruppe darstellen, die mit Fluor oder Chlor substituiert sein kann, mit Kohlenmonoxid und Wasserstoff bei erhöhter Temperatur und Druck in Gegenwart eines katalytischen Systems umgesetzt wird, das eine Rutheniumverbindung, ein Jodid und/oder Bromid eines Metalls der Gruppe II oder eine Jodid und/oder Bromid eines Übergangsmetalls und eine weitere Metallverbindung der Gruppe VIII enthält.

2. Ein Verfahren gemäß Anspruch 1, dadurch gekennzeichnet, daß als ausgangsverbindungen Verbindungen gemäß den allgemeinen Formeln $R^1$—$COOR^2$ und/oder $R^3OR^4$ verwendet werden, in denen $R^1$, $R^2$, $R^3$ und $R^4$, die gleich oder verschieden sein können, je eine Alkylgruppe mit 1 bis 12 Kohlenstoffatomen oder eine Aryl-, Alkyl-, aryl- oder Arylalkylgruppe mit bis zu 12 Kohlenstoffatomen darstellen, während $R^1$ auch ein Wasserstoffatom sein kann, und vorzugsweise gleich sind und eine Alkylgruppe mit 1 bis 12 Kohlenstoffatomen oder eine Aryl-, Alkylaryl- oder Arylalkylgruppe mit bis zu 12 Kohlenstoffatomen darstellen.

3. Ein Verfahren gemäß Anspruch 1 oder 2, dadurch gekennzeichnet, daß als Rutheniumverbindung Ruthenium(III)-chlorid, Ruthenium(III)-chlorid-trihydrat, Ruthenium(IV)-chlorid, Ruthenium(III)-bromid, Rutheniumoxid oder ein organisches Rutheniumsalz oder -komplex verwendet wird.

4. Ein Verfahren gemäß einem der vorstehenden Ansprüche, dadurch gekennzeichnet, daß als weitere Metallverbindung der Gruppe VIII eine Rhodiumverbindung verwendet wird, wie Rhodiumoxid, Rhodium(III)-hydroxid, Rhodium(III)-chlorid, Rhodium(III)-chlorid-trihydrat, Rhodium(III)-bromid, Rhodium(III)-jodid oder ein organisches Rhodiumsalz oder -komplex, vorzugsweise Rhodium(III)-chlorid-trihydrat, oder eine Palladiumverbindung, wie Palladiumchlorid, Palladiumchlorid-dihydrat, Palladiumbromid, Palladiumjodid, Palladiumoxid oder ein organisches Palladiumsalz oder -komplex, vorzugsweise Palladiumchlorid, Palladiumchlorid-dihydrat oder Palladiumacetat.

5. Ein Verfahren gemäß einem der vorstehenden Ansprüche, dadurch gekennzeichnet, daß die Rutheniumverbindung und die weitere Metallverbindung der Gruppe VIII in einem Verhältniszwischen 20:1 und 1:20 verwendet wird, vorzugsweise zwischen 5:1 und 1:5 und in einer Menge von mindestens 0,001 Gewichtsprozent und vorzugsweise in einer Menge im Bereich von 0,01 bis 10 Gewichtsprozent, bezogen auf den Carbonsäureester und/oder umzuwandelnden Äther.

6. Ein Verfahren gemäß einem der vorstehenden Ansprüche, dadurch gekennzeichnet, daß das katalytische System eine oder mehrere der Verbindungen Magnesiumbromid und/oder -jodid, Chrom(III)-jodid, Kobalt(II)-jodid, Kobalt(II)-bromid, Nickel(II)-jodid, Nickel(II)-bromid, Kupfer(II)-jodid, Zinkjodid und Zinkbromid und vorzugsweise Kobalt(II)-jodid, Zinkjodid oder Zinkbromid enthält.

7. Ein Verfahren gemäß einem der vorstehenden Ansprüche, dadurch gekennzeichnet, daß zusätzlich eine organische Phosphorverbindung, wie ein tertiäres Phosphin oder Phosphit, insbesondere Tri-n-butylphosphin oder Triphenylphosphin, eineorganische Arsenverbindung, eine organische Antimonverbindung, eine organische Stickstoffverbindung, wie Pyridine oder ein alkylsubstituiertes Pyridin, insbesondere $\alpha$-Picolin, eine organische Schwefelverbindung oder eine organische Sauerstoffverbindung in dem Reaktionsgemisch vorhanden ist, wobei die Verbindung in der Lage ist, eine Koordinationsverbindung mit dem in dem katalytischen System vorhandenen Metallrest der Gruppe VIII zu bilden.

8. Ein Verfahren gemäß einem der vorstehenden Ansprüche, dadurch gekennzeichnet, daß die Reaktion bei einer Temperatur bis zu 300°C, vorzugsweise bei einer Temperatur im Bereich von 50°C bis 200°C, und insbesondere zwischen 125°C und 175°C, durchgeführt wird.

9. Ein Verfahren gemäß einem der vorstehenden Ansprüche, dadurchgekennzeichnet, daß es unter Verwendung eines niedrigen Drucks, vorzugsweise eines Drucks zwischen 20 und 100 bar, durchgeführt wird.

10. Ein Verfahren gemäß einem der vorstehenden Ansprüche, dadurch gekennzeichnet, daß die Reaktion in Gegenwart eines Lösungsmittels durchgeführt wird, wie einer Carbonsäure, eines Carbonsäureesters, eines cyclischen Äthers, eines (a) cyclischen Sulfons, wie Dimethylsulfon, Sulfolan, 2-Methylsulfolan oder 3-Methylsulfolan, oder eines Sulfoxids, wie Dimethylsulfoxid oder Diäthylsulfoxid.

11. Ein Verfahren gemäß einem der vorstehenden Ansprüche, dadurch gekennzeichnet, daß als Ausgangsmaterial ein Carbonsäureester verwendet wird, der durch Umsetzen einer Alkancarbonsäure und eines Alkanols, insbesondere Essigsäure und Methanol, in Gegenwart eines sauren Katalysators erhalten worden ist, und daß die hergestellte Alkancarbonsäure, insbesondere Essigsäure, zu dem Ausgangsmaterial zurückgeführt wird und der erhaltene Carbonsäureester einem Umesterungsverfahren unterworfen wird.

12. Ein katalytisches System, gekennzeichnet durch eine Rutheniumverbindung, insbesondere Ruthenium(III)-chloridtrihydrat, eine weitere Metallverbindung der Gruppe VIII, insbesondere eine Rhodiumverbindung oder eine Palladiumverbindung, und ein Metalljodid und/oder -bromid der Gruppe II oder ein Übergangsmetalljodid und/oder -bromid, insbesondere Zinkjodid.

**Revendications**

1. Un procédé pour la coproduction d'acides carboxyliques des formules générales $R^1$—COOH et $R^2$—COOH et d'esters d'acides carboxyliques des formules générales $R^1$—$COOCH_2R^2$ et $R^2$—$COOCH_2R^1$, dans lesquelles $R^1$ et $R^2$, qui peuvent être identiques ou différents, représentent chacun un groupe alcoyle ayant de 1 à 20 atomes de carbone qui peut être substitué par du fluor ou du chlore ou représentent un groupe aryle, alcaryle ou aralcoyle qui peut être substitué par du fluor ou du chlore tandis que $R^1$ peut aussi représenter un atome d'hydrogène, caractérisé en ce qu'un ester d'acide carboxylique de la formule générale $R^1$—$COOR^2$ et/ou un éther de la formule générale $R^3OR^4$, dans lesquelles $R^1$ et $R^2$ sont tels que définis ci-dessus et $R^3$ et $R^4$ qui peuvent être identiques ou différents représentent chacun un groupe alcoyle ayant de 1 à 20 atomes de carbone qui peut être substitué par du fluor ou du chlore ou représentent un groupe aryle, alcaryle ou aralcoyle qui peut être substitué par du fluor ou du chlore, sont mis à réagir avec de l'oxyde de carbone et de l'hydrogène à température et pression élevées en présence d'un système catalytique qui comprend un composé du ruthénium, un iodure et/ou un bromure d'un métal du groupe II et/ou un iodure et/ou un bromure d'un métal de transition et un composé d'un autre métal du groupe VIII.

2. Un procédé selon la revendication 1, caractérisé en ce que comme matières de départ on utilise des composés des formules générales $R^1$—$COOR^2$ et/ou $R^3OR^4$ dans lesquelles $R^1$, $R^2$, $R^3$ et $R^4$ qui peuvent être identiques ou différents représentent chacun un groupe alcoyle ayant de 1 à 12 atomes de carbone ou un groupe aryle, alcaryle ou aralcoyle ayant jusqu'à 12 atomes de carbone tandis que $R^1$ peut aussi représenter aussi un atome d'hydrogène, et de préférence sont identiques et représentent un groupe alcoyle ayant de 1 à 12 atomes de carbone ou un groupe aryle, alcaryle ou aralcoyle ayant jusqu'à 12 atomes de carbone.

3. Un procédé selon la revendication 1 ou 2, caractérisé en ce que comme composé du ruthénium on utilise du chlorure de ruthénium (III), du trihydrate de chlorure de ruthénium (III), du chlorure de ruthénium (IV), du bromure de ruthénium (III), de l'oxyde de ruthénium ou un sel organique ou un complexe de ruthénium.

4. Un procédé selon l'une quelconque des revendications précédentes, caractérisé en ce que composé d'un autre métal du groupe VIII on utilise un composé du rhodium comme de l'oxyde de rhodium, de l'hydroxyde de rhodium (III), du chlorure de rhodium (III), du trihydrate de chlorure de rhodium (III), du bromure de rhodium (III), de l'iodure de rhodium (III) ou un sel organique ou un complexe de rhodium, de préférence du trihydrate de chlorure de rhodium (III), ou un composé du palladium comme du chlorure de palladium, du dihydrate de chlorure de palladium, du bromure de palladium, de l'iodure de palladium, de l'oxyde de palladium ou un sel organique ou un complexe de palladium, de préférence du chlorure de palladium, du dihydrate de chlorure de palladium ou de l'acétate de palladium.

5. Un procédé selon l'une quelconque des revendications précédentes, caractérisé en ce qu'on utilise le composé du ruthénium et le composé d'un autre métal du groupe VIII dans un rapport compris entre 20:1 et 1:20, de préférence entre 5:1 et 1:5, et à raison d'au moins 0,001% en poids et de préférence à raison de 0,01 à 10% en poids, par rapport à l'ester d'acide carboxylique et/ou à l'éther à transformer.

6. Un procédé selon l'une quelconque des revendications précédentes, caractérisé en ce que le système catalytique comprend un ou plusieurs des composés bromure et/ou iodure de magnésium, iodure de chrome (III), iodure de cobalt (II), bromure de cobalt (II), iodure de nickel (II), bromure de nickel (II), iodure de cuivre (II), iodure de zinc et bromure de zinc, et de préférence iodure de cobalt (II), iodure de zinc ou bromure de zinc.

7. Un procédé selon l'une quelconque des revendications précédentes, caractérisé en ce que de plus un composé organo-phosphore tel qu'une phosphine tertiaire ou un phosphite, spécialement la tri-n-butyl phosphine ou la triphénylphosphine, un composé organo-arsenic, un composé organo-antimoine, un composé organo-azote tel que la pyridine ou une pyridine alcoylée, spécialement l'alpha-picoline, un composé organo-soufre ou un composé organo-oxygène est présent dans le mélange réactionnel, ce composé étant capable de former un composé de coordination avec une portion métal du groupe VIII présente dans le système catalytique.

8. Un procédé selon l'une quelconque des revendications précédentes, caractérisé en ce que la réaction est conduite à une température allant jusqu'à 300°C, de préférence à une température comprise entre 50°C et 200°C, et spécialement entre 125°C et 175°C.

9. Un procédé selon l'une quelconque des revendications précédentes, caractérisé en ce qu'on met en oeuvre le procédé en utilisant une pression peu élevée, de préférence une pression comprise entre 20 et 100 bars.

10. Un procédé selon l'une quelconque des revendications précédentes, caractérisé en ce que la

réaction est conduite en présence d'un solvant tel qu'un acide carboxylique, un ester d'acide carboxylique, un éther cyclique, une sulfone acyclique ou cyclique comme la diméthylsulfone, le sulfolane, le 2-méthylsulfolane ou le 3-méthylsulfolane ou un sulfoxyde comme de diméthylsulfoxyde ou le diéthylsulfoxyde.

11. Un procédé selon l'une quelconque des revendications précédentes, caractérisé en ce que comme matière de départ on utilise un ester d'acide carboxylique que l'on a obtenu en faisant réagir un acide alcanoïque et un alcanol, spécialement l'acide acétique et le méthanol, en présence d'un catalyseur acide, et éventuellement l'acide alcanoïque produit, spécialement l'acide acétique, est recyclé à la matière de départ et l'acide carboxylique obtenu est soumis à une trans-estérification.

12. Un système catalytique caractérisé par un composé du ruthénium, spécialement le trihydrate de chlorure de ruthénium (III), un composé d'un autre métal du groupe VIII, spécialement un composé du rhodium ou un composé du palladium, et un iodure et/ou un bromure d'un métal du groupe II ou un iodure et/ou un bromure d'un metal de transition, spécialement l'iodure de zinc.